# EUROPEAN PATENT APPLICATION

(11) **EP 4 786 490 A1**
(43) Date of publication of application: **05.08.2026**
(21) Application number: 24870942.0
(22) Date of filing: 27.09.2024
(51) Int. Cl.: C07K 16/00, C12P 21/00, A61K 47/50, C12N 9/10

(54) **ENDOGLYCOSIDASE S2 MUTANT, PREPARATION METHOD THEREFOR AND USE THEREOF IN ANTIBODY GLYCOSYLATION MODIFICATION**

(30) Priority: 28.09.2023 CN 202311275618
(71) Applicant: Shanghai Tangling Biomedical Co., Ltd., Shanghai 201210 (CN); Biodlink Biopharm Co., Ltd., Suzhou, Jiangsu 215000 (CN); Shanghai Institute of Materia Medica, Chinese Academy of Sciences, Shanghai 201203 (CN)
(72) Inventor: HUANG, Wei, Shanghai 201203 (CN); YANG, Yang, Shanghai 201210 (CN); TANG, Yubo, Suzhou, Jiangsu 215000 (CN); SHI, Wei, Shanghai 201203 (CN); TANG, Feng, Shanghai 201203 (CN); ZHAO, Yan, Shanghai 201203 (CN); XIA, Fei, Shanghai 201203 (CN); ZOU, Xiangman, Shanghai 201203 (CN); HUANG, Tianxiong, Suzhou, Jiangsu 215000 (CN); ZHU, Yanyun, Suzhou, Jiangsu 215000 (CN); DUAN, Qing, Suzhou, Jiangsu 215000 (CN)
(74) Representative: Grünecker Patent- und Rechtsanwälte PartG mbB
(86) International application number: PCT/CN2024/121676
(87) International publication number: WO 2025/067404

(57) **Abstract**

An endoglycosidase S2 mutant, a preparation method therefor and the use thereof in antibody glycosylation modification. The endoglycosidase S2 mutant comprises a carbohydrate-binding module CBM defined by M677-D843 in SEQ ID NO: 1, and the amino acid sequence of the endoglycosidase S2 mutant comprises SEQ ID NO: 3, SEQ ID NO: 4 or SEQ ID NO: 5.

## Description

### Technical field

The present application belongs to the field of glycoengineering or enzyme engineering antibodies, and specifically relates to an endoglycosidase S2 mutant with antibody transglycosylation function, its preparation method and its use in antibody glycosylation modification.

### Background

The Fc glycan of IgG antibody primarily has a complex disaccharide structure with a core fucosylated double antenna. Upon co-incubation with Endo-S2 under specific conditions, the glycan at the N297 site of the antibody is hydrolyzed, exposing a GlcNAc disaccharide structure with a core fucosylate.

Endo-S2 is an endoglycosidase belonging to glycoside hydrolase family 18 (GH18). Along with its family members Endo S, Endo F1, Endo F2, and Endo F3, Endo-S2 exhibits broad-spectrum hydrolytic deglycosylation activity against glycoproteins, particularly showing significant Fc deglycosylation activity against IgG. Relevant literature reported the broad-spectrum N-glycan specificity of endoglycosidase S2 (Endo-S2) for therapeutic IgG monoclonal antibodies and the molecular basis of its processing, with the crystal structure and its hydrolytic activity of Endo-S2 being disclosed and elucidated.

According to previous reports, a series of single-amino acid mutants obtained by mutating key amino acid sites of glycoside hydrolases, such as the D233 mutant (based on Endo S) and the D184 mutant (based on Endo-S2) (see CN109071630A), exhibit significant inhibition of hydrolytic activity. CN108026518A discloses a series of Endo S mutant enzymes exhibiting as an N-linked carbohydrate that binds Asn at position 297 in IgG reduced hydrolytic activity of the activity of the chain; and CN110234341A discloses a series of Endo-S2 mutant enzymes with improved transglycosylation activity and reduced hydrolytic activity. These mutant enzymes with inhibited hydrolytic activities play a crucial role in protein/antibody glycoengineering. This finding further illustrates Endo-S and Endo-S2 exhibit high structural similarity, with their respective domains playing identical or similar roles in the catalytic reaction. Based on literature reports, Endo-S and Endo-S2 show activity differences in the recognition of different glycoform substrates, while previous reports have focused on exploring their hydrolytic activities against IgG substrates. Enzymes play a unique and crucial role in antibody glycosylation modification. Furthermore, in antibody-drug conjugation (ADC), there is great application prospect to develop enzymes with transglycosylation activity, or simultaneously possessing hydrolytic and transglycosylation activities to be used in the fields of antibody and antibody-drug conjugate by using site-directed enzyme conjugation as a third-generation technology.

### Summary of the invention

One technical objective of the present invention is to provide an endoglycosidase S2 mutant fusing different domains of Endo-S and Endo-S2, which exhibits hydrolytic activity comparable to wild-type Endo-S2, while possessing more stable transglycosylation activity compared to wild-type Endo-S2. Therefore, it can be effectively used for antibody-drug conjugation.

Another technical objective of the present invention is to provide a method for preparing the aforementioned endoglycosidase S2 mutant.

Another technical objective of the present invention is to provide a polynucleotide encoding the aforementioned endoglycosidase S2 mutant.

Another technical objective of the present invention is to provide a recombinant vector containing the aforementioned polynucleotide.

Another technical objective of the present invention is to provide a host cell transformed with the aforementioned recombinant vector.

Another technical objective of the present invention is to provide the application of the aforementioned endoglycosidase S2 mutant in antibody glycosylation modification.

A further technical objective of the present invention is to provide a method for glycosylation modification of antibodies using the aforementioned endoglycosidase S2 mutant.

In one aspect, the present invention provides an endoglycosidase S2 mutant comprising a carbohydrate-binding module CBM as defined by M677-D843 in SEQ ID NO:1, and the endoglycosidase S2 mutant comprises amino acid sequence as set forth in SEQ ID NO: 3, SEQ ID NO: 4, or SEQ ID NO: 5.

In another aspect, the present invention provides a method for preparing the above-mentioned endoglycosidase S2 mutant, the method comprises steps of:
1) ligating the target gene encoding the endoglycosidase S2 mutant of claim 1 or 2 into an expression vector (e.g., pBSYA1S1Z plasmid, pPICZα plasmid, pPIC9K plasmid, pPIC3K plasmid, pPICZA plasmid, pPIC3.5K plasmid, pET22b plasmid, pET28a plasmid, pET30a plasmid, pET32a plasmid, pGEX-6p-1 plasmid, pTrcHis plasmid, pLysS plasmid, pLysE plasmid, pBAD plasmid, pCS plasmid, pGEX-4T-1 plasmid, pET SUMO plasmid, pUC19 plasmid, pBR322 plasmid, pYES2 plasmid, pBV plasmid, pHT01 plasmid, and pHT254 plasmid) to construct a recombinant vector;
2) transforming the recombinant vector into a host cell (e.g., *Pichia pastoris* cell, *Escherichia coli* cell, *Saccharomyces cerevisiae* cell, insect ovary cell, *Bacillus subtilis* cell) to obtain a recombinant cell; and
3) expressing the target gene in the recombinant cell to obtain the endoglycosidase S2 mutant, preferably, the method further comprises step 4): isolating and purifying the endoglycosidase S2 mutant expressed in step 3).

In another aspect, the present invention provides a polynucleotide encoding the above-mentioned endoglycosidase S2 mutant, preferably, the polynucleotide comprises a sequence as set forth in SEQ ID NO: 7, SEQ ID NO: 8, or SEQ ID NO: 9.

In yet another aspect, the present invention provides a recombinant vector comprising the above-mentioned polynucleotide.

In another aspect, the present invention provides a host cell transformed with the above-mentioned recombinant vector, wherein the host cell is a prokaryotic cell or a eukaryotic cell, preferably selected from *Pichia pastoris* cell, *Escherichia coli* cell, *Saccharomyces cerevisiae* cell, insect ovary cell, and *Bacillus subtilis* cell.

In another aspect, the present invention provides use of the above-mentioned endoglycosidase S2 mutant in antibody glycosylation modification,
wherein, the glycosylation modification includes: hydrolysis of a glycosyl group in an antibody, transglycosylation of an antibody, and both,
for example, the glycosylation modification includes preparing an antibody-drug conjugate using an antibody and a disaccharide-small molecule drug conjugate in a one-step method,
wherein, the disaccharide-small molecule drug conjugate is represented by Formula III below: 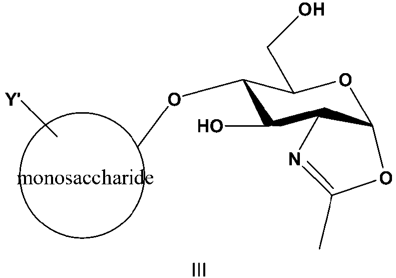 in Formula III, 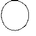 represents a monosaccharide selected from galactose, mannose, and glucose, with its C5 position replaced by Y', and the bond between the two monosaccharides is a 1,4 glycosidic bond,
Y' is represented by 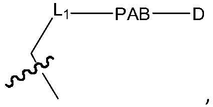
wherein, L₁ represents a dipeptide linker, for example, -Val-Cit-, -Val-Ala-;
PAB represents a p-aminophenylethanol linker;
D represents a small molecule drug moiety, for example, D may be MMAE, MMAF, or maytansine;
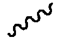 indicates the linking position with the monosaccharide,
preferably, the disaccharide-small molecule drug conjugate has the following structure: 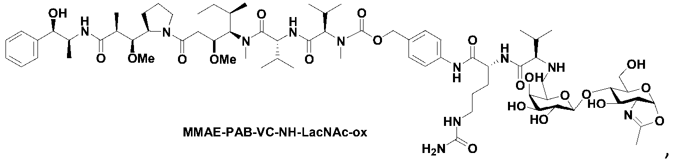
alternatively, the glycosylation modification comprises: preparing an antibody-drug conjugate by a two-step method using an antibody and a sugar substrate of Formula II below: 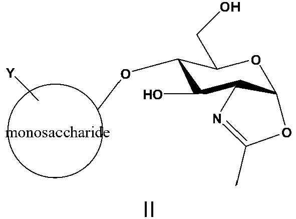 in Formula II, 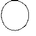 represents a monosaccharide selected from galactose, mannose, and glucose, with its C5 position replaced by Y,
wherein Y is selected from: 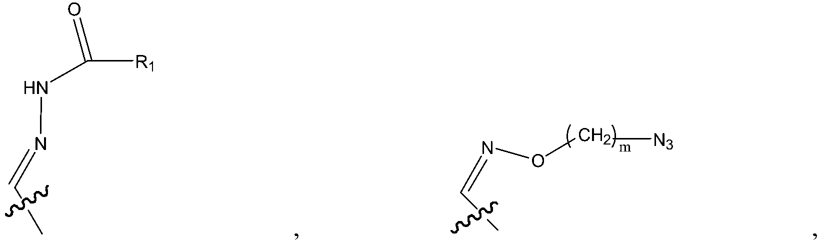 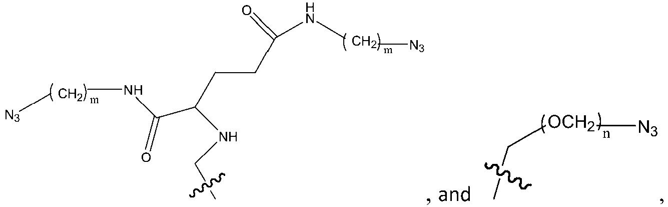
wherein R₁ represents C1-C7 alkyl, preferably C2-C6 alkyl, preferably C3-C5 alkyl, and most preferably C3-C4 alkyl, such as methyl, ethyl, propyl, butyl, pentyl, hexyl, and heptyl;
m represents an integer from 1 to 6, for example, m is 1, 2, 3, 4, 5, or 6;
n represents an integer from 1 to 24, for example, n is 1, 2, 3, 4, 5, 6, 7, 8, 9, 10, 11, 12, 13, 14, 15, 16, 17, 18, 19, 20, 21, 22, 23, or 24;
L₁ represents a dipeptide linker, for example, -Val-Cit- or -Val-Ala-;
PAB represents a p-aminophenylethanol linker;
D represents a small molecule drug moiety, for example, D may be MMAE or MMAF;
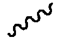 indicates the linking position with the monosaccharide.

In another aspect, the present invention provides a method for glycosylation modification of an antibody, the method comprises the step of using the aforementioned endoglycosidase S2 mutant during a glycosylation modification process.

In a specific embodiment, the antibody is an antibody having a glycosylation site.

In a specific embodiment, the antibody is an antibody having an N-glycosylation site.

In a specific embodiment, the antibody is a bi-antennary antibody having an N-glycosylation site. In a specific embodiment, the antibody is IgG with a conserved N-glycosylation site at N297 in the Fc region.

In a specific embodiment, the antibody is a monoclonal antibody, a polyclonal antibody, a bifunctional antibody, a trifunctional antibody, a nanobody fused with an Fc domain, a therapeutic antibody or a functional antibody from a different species.

Specifically, the antibody is a human antibody, a murine antibody, or a chimeric antibody; specifically, the antibody is IgG1, IgG2, or IgG4; in a specific embodiment, the targets of the antibody include HER2, Claudin 18.2, EGFR, c-Met, NECTIN4, CD276, HER3, CD3, FOLR1, BCMA, CD20, DLL3, MUC1, PD-L1, ROR1, TF, CD19, CD22, CD30, CD70, CD79B, FGFs, MSLN, NT5E, TNFα, CD147, CD24, CD38, CD47, CDH3, CDK4, CDK6, CEACAM5, CLDN6, CTLA4, DDR1, and D. R5, FAPα, FGFR3, GPRC5D, GR, HLA-DR, ICAM1, IL2R, MELTF, ROR2, TPBG(5T4), VTCN1, ZIP6, CD33, CD25, RSV, VEGF, RANKL, VEGFR2, CTLA-4, CD52, CD319, PD-1, CD274, IgE, IL-6, IL-12, IL-2, C5, IL-17A, CD25, SLAMF7, F10, factor IXa, HAb18G, PCSK9, BlyS, IL23, α4β7, IL-4R-α, HAE, FGF23, and IL6R; preferably, the targets of the antibodies include HER2, CD20, EGFR, and PD-1;
in a specific embodiment, the antibodies include trastuzumab, rituximab, pertuzumab, panitumumab, toripalimab, and nivolumab.

In a specific embodiment, the method comprises steps of:
1) incubating an antibody with the aforementioned endoglycosidase S2 mutant to hydrolyze the glycosyl group on the antibody ; and/or
2) performing glycosylation modification on the antibody using a sugar substrate under the transglycosylation action of the endoglycosidase S2 mutant,
   wherein,
   the sugar substrate is a disaccharide represented by Formula I below: 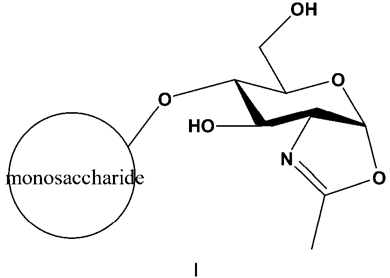
   wherein, 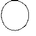 represents a monosaccharide selected from galactose, mannose, and glucose, and
   the bond between the two monosaccharides is a 1,4 glycosidic bond;
      or
   the sugar substrate is a disaccharide derivative represented by Formula II below: 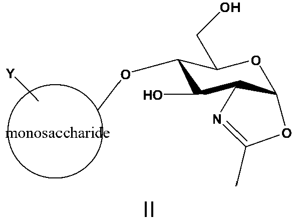 in Formula II, 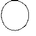 represents a monosaccharide selected from galactose, mannose, and glucose,
   with its C5 position replaced by Y,
   wherein Y is selected from: 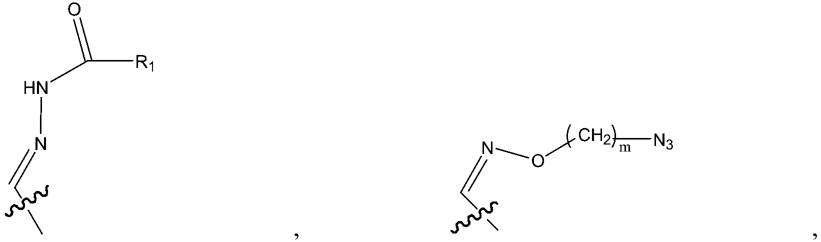 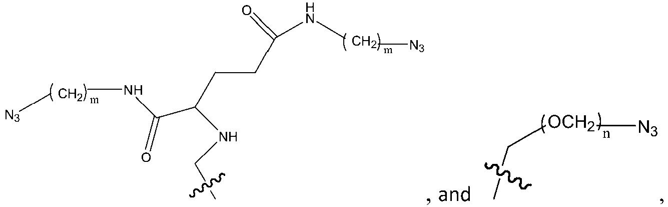
   wherein R₁ represents C1-C7 alkyl, preferably C2-C6 alkyl, preferably C3-C5 alkyl, and preferably C3-C4 alkyl, such as methyl, ethyl, propyl, butyl, pentyl, hexyl, and heptyl;
   m represents an integer from 1 to 6, for example, m is 1, 2, 3, 4, 5, or 6;
   n represents an integer from 1 to 24, for example, n is 1, 2, 3, 4, 5, 6, 7, 8, 9, 10, 11, 12, 13, 14, 15, 16, 17, 18, 19, 20, 21, 22, 23, or 24;
   L₁ represents a dipeptide linker, for example, -Val-Cit- or -Val-Ala-;
   PAB represents a p-aminophenylethanol linker;
   D represents a small molecule drug moiety, for example, D may be MMAE or MMAF;
   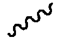 indicates the linking position with the monosaccharide;
   alternatively,
   the sugar substrate is a disaccharide-small molecule drug conjugate represented by Formula III below: 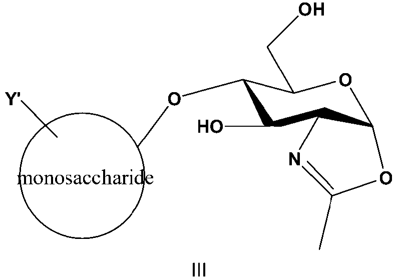 in Formula III, 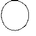 represents a monosaccharide selected from galactose, mannose, and glucose,
   with its C5 position replaced by Y',
   Y' is represented by 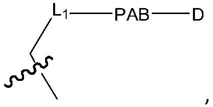
   wherein, L₁ represents a dipeptide linker, for example, -Val-Cit-, -Val-Ala-;
   PAB represents a p-aminophenylethanol linker;
   D represents a small molecule drug moiety, for example, D may be MMAE or MMAF;
   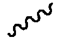 indicates the linking position with the monosaccharide.

Specifically, the sugar substrate is selected from the following:

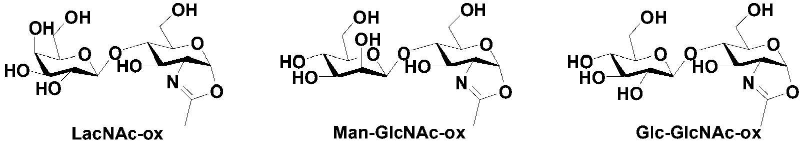

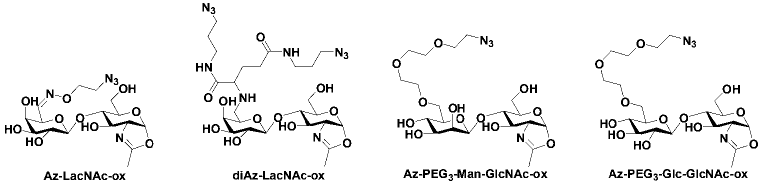

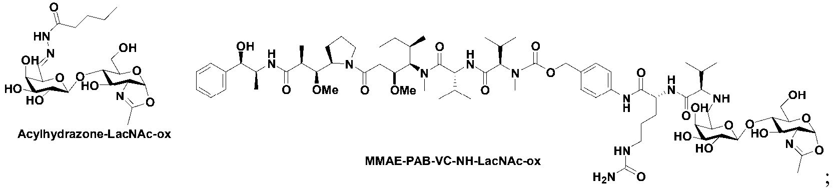

Alternatively,
the sugar substrate is a sugar substrate of the above Formula I, II, or III, of which their oxazoline ring is in an open-ring form;
preferably, the method further comprises the step of reacting the glycosylated antibody modified with the sugar substrate represented by Formula II with a drug-linker to prepare an antibody-drug conjugate, preferably, the drug-linker contains a functional group of hydroxylamine, such as the drug-linker is NH₂O-VC-PAB-MMAE.

### Advantageous effects

The endoglycosidase S2 mutants S2M3 and V3 obtained by fusing different domains of Endo-S and Endo-S2 in the present invention exhibit hydrolytic activity comparable to that of wild-type Endo-S2.

The endoglycosidase S2 mutants S2M3 and V3 obtained in the present invention can be used for antibody glycosylation modification, showing good transglycosylation activity for various glycoforms such as LacNAc-ox, Man-GlcNAc-ox, Glc-GlcNAc-ox, and their derivatives, among them, S2M3 exhibits more stable transglycosylation activity compared to Endo-S2.

The endoglycosidase S2 mutants S2M3 and V3 obtained in the present invention can be effectively used for antibody drug conjugation, thus enabling the preparation of antibody-drug conjugates (ADCs).

### Description of drawings

Figure 1 shows the hydrolysis results of the sugar chain at the N-glycosylation site of trastuzumab under the action of Endo-S2, S2M1, S2M3, S2M4, and V3. Figure A shows the hydrolysis of the sugar chain at the N-glycosylation site of the antibody after co-incubating trastuzumab with each enzyme for 1 hour, and Figure B shows the hydrolysis of the sugar chain at the N-glycosylation site of the antibody after co-incubating trastuzumab with each enzyme for 4 hours.
Figure 2 shows the transglycosylation activity test results of endoglycosidases using Acylhydrazone-LacNAc-ox as the sugar substrate, in which four mutant enzymes were tested.
Figure 3 shows the RP test results (A) and LC-MS (B) test results of the antibody-drug conjugate (ADC) prepared in Example 4.
Figure 4 shows the transglycosylation activities of S2M1, S2M4, and V3 after increasing the sugar substrate equivalent.
Figure 5 shows the transglycosylation activity assay results of mutant enzymes (S2M1, S2M3, S2M4, V3) using MMAE-PAB-VC-NH-LacNAc-ox as the sugar substrate, performed in a one-step method.
Figure 6 shows the effect of the amount of Endo-S2 and S2M3 enzymes on the transglycosylation activity toward the antibody using Az-LacNAc-ox.
Figure 7 shows the effect of the amount of Endo-S2 and S2M3 enzymes on the transglycosylation activity toward the antibody using diAz-LacNAc-ox.
Figure 8 shows the effect of reaction time on the transglycosylation activity toward the antibody using diAz-LacNAc-ox , at different amounts of Endo-S2 and S2M3 enzymes, respectively.

### Detailed embodiments

The technical solutions of the present application are described in detail below by specific embodiments, such that those skilled in the art can better understand the present application; however, it should be understood that the scope of the present application is not limited to these embodiments.

### Term

In the present application, "endoglycosidase S2 mutant," "novel endoglycosidase," and "mutant enzyme" can be used interchangeably.

In the present application, the monosaccharides at the glycosylation site of an antibody are represented by the following symbols: 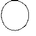 galactose, 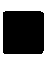 ; N-acetylglucosamine, 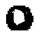 ; mannose, and 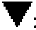 fucose.

In the present application, the following sugar substrates: Az-LacNAc-ox, diAz-LacNAc-ox, Az-PEG₃-Man-GlcNAc-ox, Az-PEG₃-Glc-GlcNAc-ox, Acylhydrazone-LacNAc-ox, and MMAE-PAB-VC-NH-LacNAc-ox, which were used for testing the transglycosylation activity of the novel endoglycosidases, were custom-made:

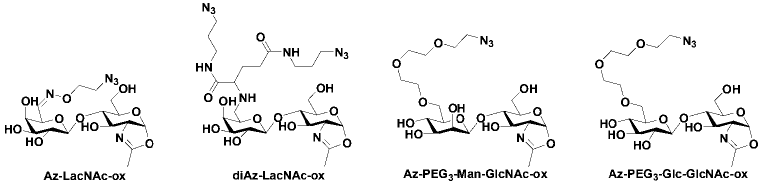

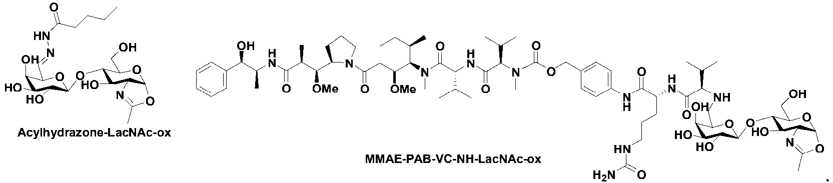

In the present application, the drug-linker NH₂O-VC-PAB-MMAE was custom-made.
Az-LacNAc-ox was G3 in CN114949236A.
diAz-LacNAc-ox was G14 in CN114949236A.
Az-PEG₃-LacNAc-ox was commercially available.
Az-PEG₆-LacNAc-ox was commercially available.
Az-PEG₁₂-LacNAc-ox was commercially available.
Az-PEG₂₄-LacNAc-ox was commercially available.
Az-PEG₃-Man-GlcNAc-ox was commercially available.
Az-PEG₃-Glc-GlcNAc-ox was commercially available.
Acylhydrazone-LacNAc-ox was commercially available.
NH₂O-VC-PAB-MMAE was the compound D1 in CN114949236A.

### General Operation 1: Expression and Purification of Endoglycosidase

The target gene (SEQ ID NO: 7/ SEQ ID NO: 8/ SEQ ID NO: 9/ SEQ ID NO: 10) was ligated to the pET22b(+) vector to construct a plasmid. The plasmid was transformed into *E. coli* DH5α competent cells for cloning. 1 µL of the constructed plasmid (10 ng/µL) was transformed into 50 µL of *E. coli* DH5α competent cells and incubated on ice for 30 min. The cells were then heat-shocked in a 42°C water bath for 90 sec, and then transferred to ice and incubated for 3-5 min. 500 µL of antibiotic-free LB medium was added thereto, the resultant was mixed well, and the cells were recovered at 37°C with shaking at 220 rpm for 1-2 h. Subsequently, 200-300 µL of the bacterial culture was spread onto a LB agar plate containing ampicillin and the plate was inverted in a constant temperature incubator for incubation overnight at 37°C. The following day, a single clone was picked and cultured in 3-5 mL of LB medium. When the OD₆₀₀ reached 0.6-0.8, 50 µL of the bacterial culture was taken and transferred to the sequencing company for Sanger sequencing.

Once the sequencing results were confirmed to be correct, the plasmid was transformed into *E*. *coli* BL21(DE3) competent cells, and IPTG was used to induce the expression of endoglycosidase. 1 µL of plasmid (10 ng/µL) was transformed into 50 µL of *E. coli* BL21(DE3) competent cells and incubated on ice for 30 min. The cells were heat shocked in a 42°C of water bath for 90 sec, followed by incubation on ice for 3-5 min. After adding 500 µL of antibiotic-free LB medium and mixing well, the cells were recovered at 37°C with shaking at 220 rpm for 1-2 h. Subsequently, 200-300 µL of the bacterial culture was spread onto a LB agar plate containing ampicillin and the plate was inverted in a constant temperature incubator for incubation overnight at 37°C. The following day, a single clone was picked and cultured in 3-5 mL of LB medium. When the OD₆₀₀ reached 0.6-0.8, IPTG was added to the bacterial culture to a final concentration of 0.5 mM, and the culture was incubated overnight at 20°C and 200 rpm for expression. After expression, the culture was centrifuged at 4500 rpm for 20 min, and the supernatant was discarded to collect the cells. The cells were resuspended in 30 mL of phosphate buffer (0.01 M, pH 7.4), sonicated, and then centrifuged at 12000 rpm for 15 min to collect the supernatant for protein purification.

The overexpressed endoglycosidase was purified using a nickel affinity chromatography column. The column bed was equilibrated with 5 column volumes of phosphate buffer (0.01 M, pH 7.4), and the above supernatant obtained after cell lysis and centrifugation was then loaded into the chromatography column. This process was repeated three times to ensure the target protein bound to the column packing material. The column was eluted with 10 column volumes of 50 mM, 100 mM, and 250 mM imidazole, respectively, and the eluent was collected in separate tubes.

The eluent was subjected to polyacrylamide gel electrophoresis to determine the sample location, the sample was concentrated by ultrafiltration, and the buffer therein was replaced with phosphate buffer (0.01 M, pH 7.4).

Protein concentration was determined using the UV280 method, and molecular weight was identified by LC-MS.

**General Operation 2**: Specific Antibody N-glycan Hydrolysis Activity Assay of the Endoglycosidase.

Trastuzumab (5 mg/mL) was added to phosphate buffer (pH 7.0), followed by the addition of the endoglycosidase (100 ng/mg). The system was incubated at 30 °C for 1 hour and 4 hours, and then the resultant was sampled, analyzed and identified by LC-MS.

### General Operation 3: Glycosylation of an Antibody with the Endoglycosidases

The antibody (5 mg/mL) and oxazoline (0.3-2 mM) (also referred to as "ox" in the present application) were added to phosphate buffer (pH 6.5-7.0). After adjusting the pH of the solution to 6.5-7.0, the endoglycosidase was added (0.1-0.8 mg/mL). The system was incubated at 30 °C for 2 hours, and then the resultant was analyzed and identified by SDS-PAGE or LC-MS.

The following operations in the present application all referred to the General Operations 1-3 above.

### Example 1: Modification of Endoglycosidase Mutants

Four mutants, named V3, S2M1, S2M3, and S2M4, were obtained by combining and fusing different domains of wild-type Endo-S2 and Endo S, as shown in Table 1 below.

**Table 1: Structural Information of Novel Endoglycosidases**

| Name of Endoglycosidase/mutant | Sequence |
|---|---|
| Endo-S2 | SEQ ID NO: 1 |
| Endo-S | SEQ ID NO: 2 |
| S2M3 | SEQ ID NO: 3 |
| S2M4 | SEQ ID NO: 4 |
| V3 | SEQ ID NO: 5 |
| S2M1 | SEQ ID NO: 6 |

### Example 2: Expression and Purification of Novel Endoglycosidases Designed in Example 1

Novel endoglycosidases V3, S2M1, S2M3, and S2M4 were obtained by General Operation 1 for expression and purification.

### Example 3: Antibody Glycosylation Hydrolysis Activity Test on Novel Endoglycosidases.

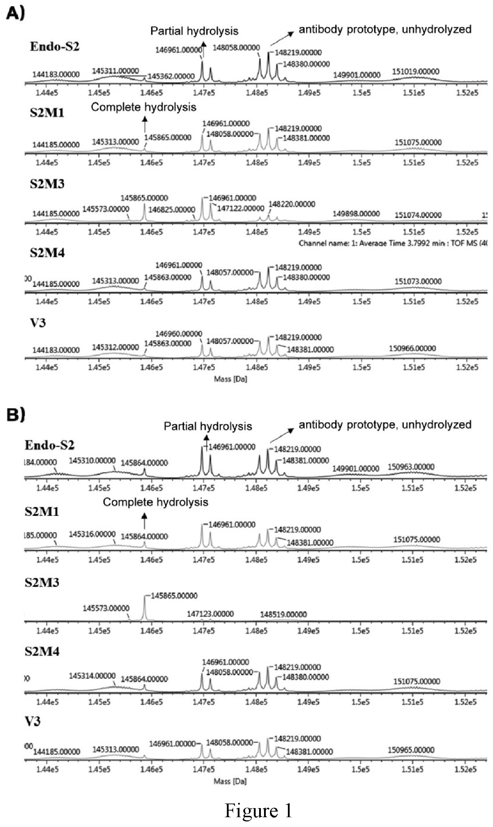

Antibody glycosylation hydrolysis activities of novel endoglycosidases V3, S2M1, S2M3, and S2M4 were tested by General Operation 2. The results are shown in Figure 1. The results showed that all the modified mutant enzymes could hydrolyze wild-type antibody sugar chains. S2M3 exhibited better hydrolysis activity than Endo-S2, while the hydrolysis activities of S2M1, S2M4, and V3 were comparable to that of Endo-S2.

### Example 4: Transglycosylation of antibody with Acylhydrazone-LacNAc-ox under the action of Novel Endoglycosidases and A Two-Step method for preparing glycosite-specific ADC Compounds

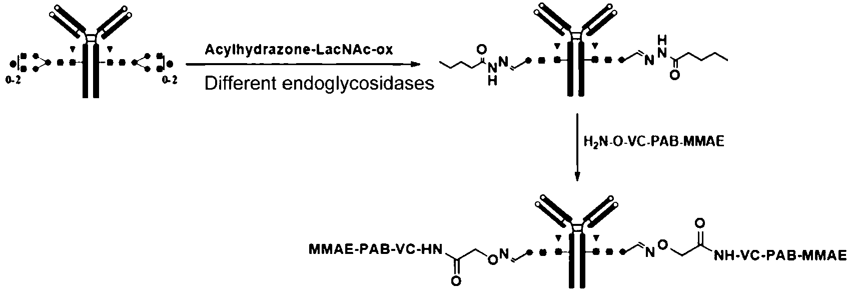

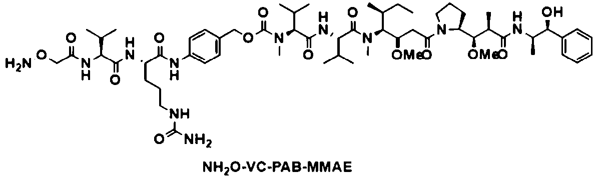

Step 1: Acylhydrazone-LacNAc-ox (0.5 mM) was added to phosphate buffer (pH 6.5). Wild-type Herceptin (5 mg/mL) and endoglycosidase (40 µg/mg, S2M1, S2M3, S2M4, and V3, respectively) were added sequentially to the above reaction system, and the resulting mixture was incubated at 30°C for 2 hours. Mass spectrometry results showed that S2M3 exhibited the best transglycosylation activity for Acylhydrazone-LacNAc-ox, with two sugar substrates coupled in a high ratio; V3 showed weaker transglycosylation activity than S2M3, while S2M1 and S2M4 showed poor transglycosylation activity.

(Purification): Excess Endo-S2 and the sugar substrate were removed from the sample using AKTA Pure 150 chromatography (Merck VL 11×250 column; Mabselect Sure XL packing material 2 mL).

Step 2: To the antibody solution modified with the sugar substrate obtained in Step 1, 50 mM NaOAc buffer (pH 4.6) was added, and DMSO (10% V/V) was added, and then 10 mg/mL H₂NO-VC-PAB-MMAE drug linker intermediate solution was added. The system was incubated at 30°C for 2 hours.

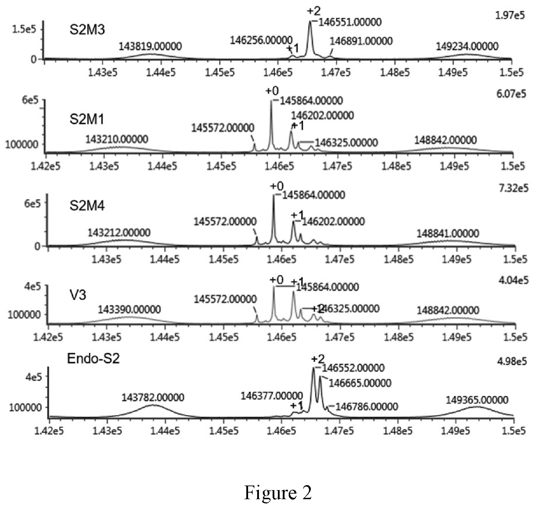

Figure 2 shows the transglycosylation activity test results of the endoglycosidases using Acylhydrazone-LacNAc-ox as the sugar substrate. Four mutant enzymes were tested. As shown in Figure 2, S2M3 exhibited the best transglycosylation activity for Acylhydrazone-LacNAc-ox, with two sugar substrates coupled in a high ratio; V3 had weaker transglycosylation activity than S2M3, while S2M1 and S2M4 had poor transglycosylation activity.

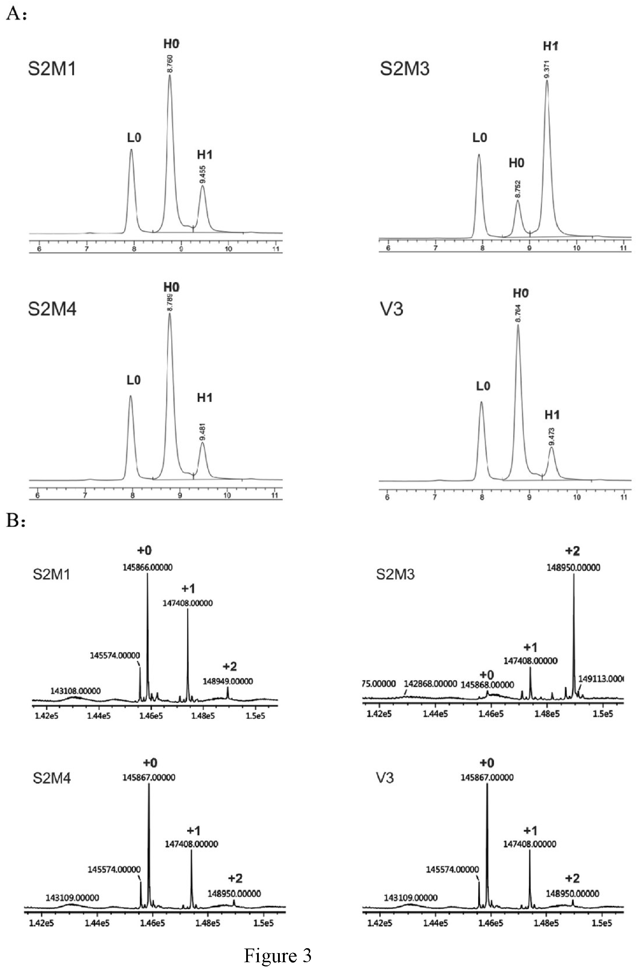

The ADC compounds as prepared above were analyzed by RP and LC-MS: the results are shown in Table 2 below and Figure 3.

**Table 2:**

| No. | Sample | RP result LC-MS result | |
|---|---|---|---|
| | | DAR value | DAR value |
| 1 | Two step method-S2M1 | 0.48 | 0.54 |
| 2 | Two step method -S2M3 | 1.61 | 1.68 |
| 3 | Two step method -S2M4 | 0.38 | 0.42 |
| 3 | Two step method -V3 | 0.38 | 0.42 |

### Example 5: Transglycosylation Activities of S2M1, S2M4, and V3 at Increased Sugar Substrate Equivalent

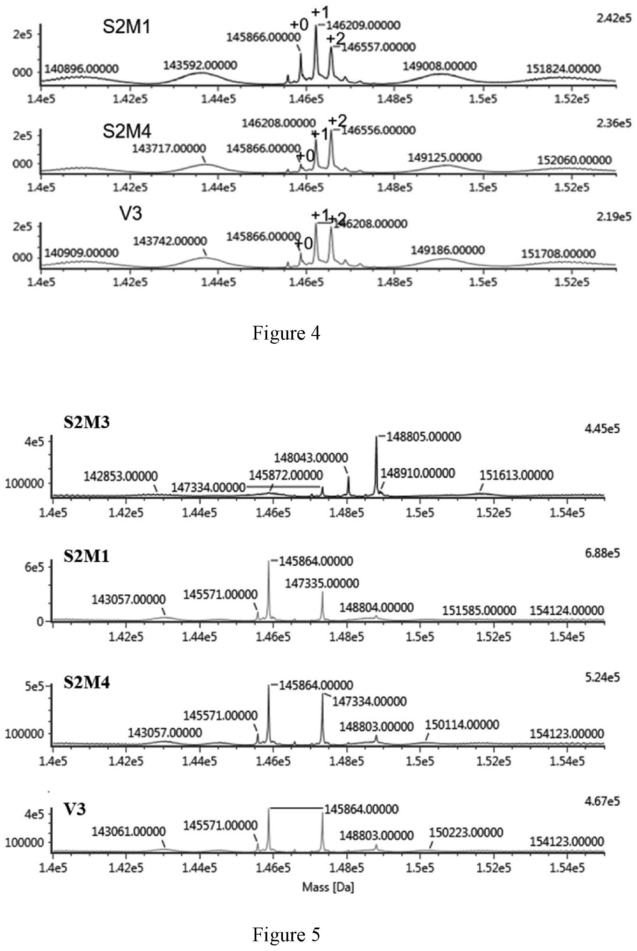

Transglycosylation activities of S2M1, S2M4, and V3 were tested in the same manner as that in Example 4, except that the equivalent amount of the sugar substrate was increased to 60 relative to wild-type Herceptin, and the transglycosylation effect was measured. The results are shown in Figure 4. As can be seen from the figure, after increasing the sugar substrate equivalent, all three enzymes, S2M1, S2M4, and V3, exhibited certain transglycosylation activities. S2M4 was superior to V3, and V3 was superior to S2M1. Therefore, S2M4 and V3, which contain the CBM region of Endo-S2, showed superior transglycosylation activities.

### Example 6: One-step Method for Preparing Glycosite-specific ADC Compounds Using Novel Endoglycosidases

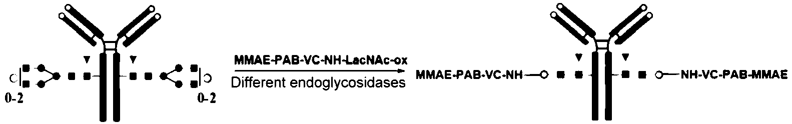

MMAE-PAB-VC-NH-LacNAc-ox (0.5 mM) was added to phosphate buffer (pH 6.5). Wild-type Herceptin (5 mg/mL) and endoglycosidase (80 µg/mg, S2M1, S2M3, S2M4, and V3, respectively) were added sequentially to the above reaction system, and incubated at 30°C for 2 hours. The results are shown in Figure 5.

S2M3 exhibited the best transglycosylation activity. S2M1, S2M4, and V3 all showed some transglycosylation activity, with S2M4 showing better activity than V3, and V3 better than S2M1. This indicated that S2M3, S2M4, and V3, which contain the CBM region of Endo-S2, exhibited superior transglycosylation activity.

### Example 7: Test to evaluate the effect of the amount of Endo-S2 and S2M3 on the antibody transglycosylation activity with Az-LacNAc-ox substrate

Az-LacNAc-ox (0.5 mM, structural formula shown below) was added to phosphate buffer (pH 6.5). Trastuzumab (5 mg/mL) and endoglycosidase (Endo-S2 or S2M3, 0.02-2.4 mg/mL) were added sequentially to the above reaction system. The system was incubated at 30 °C for 2 hours and then the resultant was analyzed and identified by LC-MS.

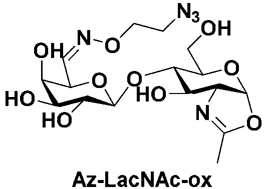

The specific parameters are shown in Table 3 below:

**Table 3. Reaction conditions in the Antibody transglycosylation activity assay using Az-LacNAc-ox substrate at different amountsof Endo-S2 and S2M3**

| **Amount of the enzyme (**µ**g/mg)** | **4, 20, 40, 80, 160, 240, 320, 480** |
|---|---|
| Enzyme | Endo-S2, S2M3 |
| Sugar substrate | Az-LacNAc-ox |
| Reaction concentration (mg/mL) | 5 |
| Ratio of LacNAc-ox to mAb | 15 |
| Reaction system | pH 6.5 |
| Reaction temperature (°C) | 30 |
| Reaction time | 2 h |

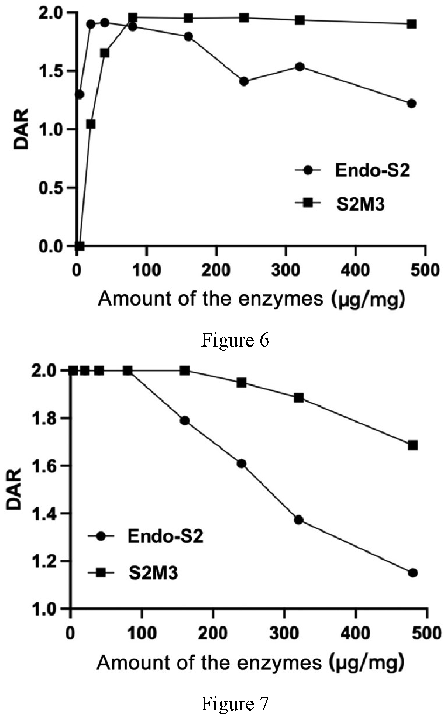

Table 3 shows the reaction conditions in the antibody transglycosylation assay at Endo-S2 or S2M3 amounts of 4, 20, 40, 80, 160, 240, 320, and 480 µg/mg (enzyme/antibody). The trastuzumab concentration was 5 mg/mL, the Az-LacNAc-ox sugar substrate concentration was 0.5 mM, and the reaction was carried out in a 50 mM phosphate buffer, pH 6.5 system. After incubating at 30 °C for 2 hours, the resultant was analyzed by LC-MS, and the results are shown in Figure 6.

The results showed that under low enzyme amount conditions (approximately <50 µg/mg), both wild-type Endo-S2 and the mutant enzyme S2M3 had a positive promoting effect on DAR values. Under higher enzyme amount, the DAR value decreased as Endo-S2 amount increased, whereas for S2M3 a longer plateau phase was shown, and high DAR values were maintained across a wide range (approximately 100-500 µg/mg). This indicates that the mutant enzyme S2M3 possesses more stable transglycosylation activity compared to Endo-S2.

### Example 8: Test to evaluate the effect of the amount of Endo-S2 and S2M3 on the antibody transglycosylation acitivity with diAz-LacNAc-ox substrate

diAz-LacNAc-ox (0.5 mM, structural formula shown) was added to phosphate-HCl buffer (pH 6.5). Trastuzumab (5 mg/mL) and endoglycosidase (0.02-2.4 mg/mL) were added sequentially to the reaction system. The system was incubated at 30 °C, and the resultant was sampled at different time points for LC-MS analysis.

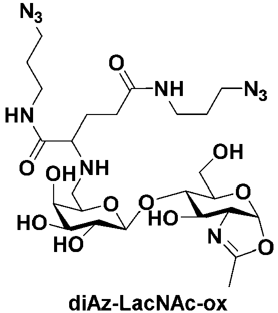

The specific parameters are shown in Table 4 below:

**Table 4. Reaction conditions in the Antibody transglycosylation activity assay using diAz-LacNAc-ox substrate at different amounts of Endo-S2 and S2M3**

| **Amount of the enzyme (µg/mg)** | **4, 20, 40, 80, 160, 240, 320, 480** |
|---|---|
| Enzyme | Endo-S2, S2M3 |
| Sugar substrate | diAz-LacNAc-ox |
| Reaction concentration (mg/mL) | 5 |
| Ratio of LacNAc-ox to mAb | 15 |
| Reaction system | pH 6.5 |
| Reaction temperature (°C) | 30 |
| Reaction time | 2h |

Table 4 shows the reaction conditions in the antibody transglycosylation activity assay at Endo-S2 or S2M3 enzyme amounts of 4, 20, 40, 80, 160, 240, 320, and 480 µg/mg (enzyme/antibody ). The trastuzumab concentration was 5 mg/mL, the diAz-LacNAc-ox sugar substrate concentration was 0.5 mM, and the reaction was carried out in a 50 mM phosphate buffer, pH 6.5, at 30 °C. The resultant was sampled at different time points for LC-MS analysis, as shown in Figure 7.

The results showed that with prolonged reaction time, at all enzyme amounts, the mutant enzyme S2M3 exhibited lower and slower hydrolytic activity than Endo-S2, which is more beneficial for process production.

### Experimental Result Analysis:

It can be seen from the results of Examples 5-8 that, the mutant enzyme S2M3 is significantly superior to Endo-S2 in antibody transglycosylation reactions with different sugar substrates. The reaction curve of mutant S2M3 exhibited distinct enzymatic reaction characteristics, maintaining a stable plateau phase and consistently high DAR values across a wide enzyme amount range. This indicated that after hydrolyzing the antibody Fc sugar chains, mutant enzyme S2M3 efficiently transfered the Az-LacNAc-ox sugar substrate to the antibody, showing no hydrolytic activity on the transglycosylated glycan-engineered antibody. This efficient and stable transglycosylation activity makes such mutant enzyme exhibit dosage robustness in the preparation of antibody conjugates. On the one hand, the longer plateau phase weakens the influence of enzyme dosage on the DAR value of the product; on the other hand, the reduced substrate hydrolytic activity further highlights the enhanced transglycosylation activity of the mutant enzyme. In conclusion, this mutant enzyme has superior practical application value in processes.

**Example 9**: Test to evaluate the effect of reaction time on the antibody transglycosylation acitivity with diAz-LacNAc-ox substrate at the same amount of Endo-S2 and S2M3.

The test was performed in the same manner as in Example 6, with details shown in Table 5 below. The effect of reaction time on antibody transglycosylation activity with diAz-LacNAc-ox substrate was investigated at the same amount of Endo-S2 and S2M3.

**Table 5: Reaction conditions in the test to evaluate the effect of amount of Endo-S2 and S2M3 on the antibody transglycosylation activity using diAz-LacNAc-ox substrate**

| **Amount of the enzyme (µg/mg)** | 40, 80, 160, 240 |
|---|---|
| Enzyme | Endo-S2, S2M3 |
| Sugar substrate | diAz-LacNAc-ox |
| Reaction concentration (mg/mL) | 5 |
| Ratio of LacNAc-ox to mAb | 15 |
| Reaction system | pH 6.5 |
| Reaction temperature (°C) | 30 |
| Reaction time | 15min, 30min, 45min, 1h, 1.5h, 2h, 4h, 8h |

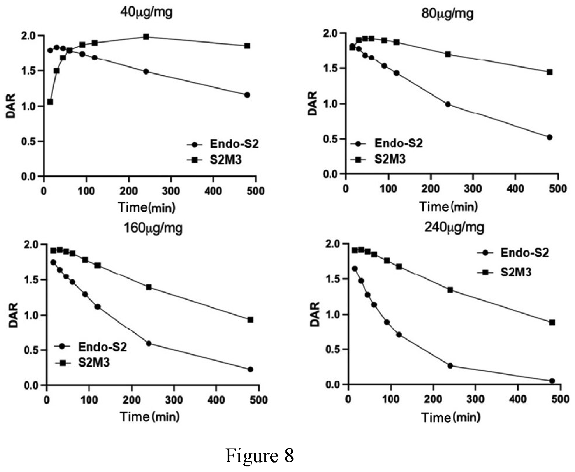

The results are shown in Figure 8. As can be seen from Figure 8, with prolonged reaction time, at various enzyme amounts, the mutant enzyme S2M3 exhibited lower and slower hydrolytic activity compared to Endo-S2, thus S2M3 is more beneficial for the production process.

Non-natural glycoengineered antibodies were prepared by using General Operation 3 with different antibodies and different sugar substrates as raw materials as below.

### Example 10: Synthesis of Ab-1

The non-natural glycoengineered antibody Ab-1 was obtained by using the compound Az-LacNAc-ox and the wild-type antibody Rituximab (Human IgG1) via General Operation 3 (S2M3 enzyme). The HRMS value after deconvolution was 145780.

### Example 11: Synthesis of Ab-2

The non-natural glycoengineered antibody Ab-2 was obtained by using the compound Az-LacNAc-ox and the wild-type antibody Pertuzumab (Human IgG1) via General Operation 3 (S2M3 enzyme). The HRMS value after deconvolution was 146791.

### Example 12: Synthesis of Ab-3

The non-natural glycoengineered antibody Ab-3 was obtained by using the compound Az-LacNAc-ox and the wild-type antibody panitumumab (Human IgG2) via General Operation 3 (S2M3 enzyme). The HRMS value after deconvolution was 145631.

### Example 13: Synthesis of Ab-4

The non-natural glycoengineered antibody Ab-4 was obtained by using the compound Az-LacNAc-ox and the wild-type antibody toripalimab (Human IgG4) via General Operation 3 (S2M3 enzyme). The HRMS value after deconvolution was 148616.

### Example 14: Synthesis of Ab-5

The non-natural glycoengineered antibody Ab-5 was obtained by using the compound Az-LacNAc-ox and the wild-type antibody nivolumab (Human IgG4) via General Operation 3 (S2M3 enzyme). The HRMS value after deconvolution was 144929.

### Example 15: Synthesis of Ab-6

The non-natural glycoengineered antibody Ab-6 was obtained by using compound Az-LacNAc-ox and murine monoclonal antibody-1 via General Operation 3 (S2M3 enzyme). The HRMS value after deconvolution was 146927.

### Example 16: Synthesis of Ab-7

The non-natural glycoengineered antibody Ab-7 was obtained by using compound Az-LacNAc-ox and murine monoclonal antibody-2 via General Operation 3 (S2M3 enzyme). The HRMS value after deconvolution was 147312.

### Example 17: Synthesis of Ab-8

The non-natural glycoengineered antibody Ab-8 was obtained by using compound Az-LacNAc-ox and murine monoclonal antibody-3 via General Operation 3 (S2M3 enzyme). The HRMS value after deconvolution was 146550.

### Example 18: Synthesis of Ab-9

The non-natural glycoengineered antibody Ab-9 was obtained by using the compound Man-GlcNAc-ox and trastuzumab via General Operation 3 (S2M3 enzyme). The HRMS value after deconvolution was 146590.

### Example 19: Synthesis of Ab-10

The non-natural glycoengineered antibody Ab-10 was obtained by using the compound Glc-GlcNAc-ox and trastuzumab via General Operation 3 (S2M3 enzyme). The HRMS value after deconvolution was 146591.

### Example 20: Synthesis of Ab-11

The non-natural glycoengineered antibody Ab-11 was obtained by using the compound Az-PEG₃-Man-GlcNAc-ox and trastuzumab via General Operation 3 (S2M3 enzyme). The HRMS value after deconvolution was 146904.

### Example 21: Synthesis of Ab-12

The non-naturally glycoengineered antibody Ab-12 was obtained by using the compound Az-PEG₃-Glc-GlcNAc-ox and trastuzumab via General Operation 3 (S2M3 enzyme). The HRMS value after deconvolution was 146904.

### Example 22: Synthesis of Ab-13

The non-naturally glycoengineered antibody Ab-13 was obtained by using the compound Az-PEG₃-LacNAc-ox and trastuzumab via General Operation 3 (S2M3 enzyme). The HRMS value after deconvolution was 146996.

### Example 23: Synthesis of Ab-14

The non-naturally glycoengineered antibody Ab-14 was obtained by using the compound Az-PEG₆-LacNAc-ox and trastuzumab via General Operation 3 (S2M3 enzyme). The HRMS value after deconvolution was 147260.

### Example 24: Synthesis of Ab-15

The non-natural glycoengineered antibody Ab-15 was obtained by using the compound Az-PEG₁₂-LacNAc-ox and trastuzumab via General Operation 3 (S2M3 enzyme). The HRMS value after deconvolution was 147784.

### Example 25: Synthesis of Ab-16

The non-natural glycoengineered antibody Ab-16 was obtained by using the compound Az-PEG₂₄-LacNAc-ox and trastuzumab via General Operation 3 (S2M3 enzyme). The HRMS value after deconvolution was 148840.

## Claims

1. An endoglycosidase S2 mutant comprising a carbohydrate-binding module CBM as defined by M677-D843 in SEQ ID NO:1, and the endoglycosidase S2 mutant comprises amino acid sequence as set forth in SEQ ID NO: 3, SEQ ID NO: 4, or SEQ ID NO: 5.

2. A method for preparing the endoglycosidase S2 mutant of claim 1, the method comprises steps of:
1) ligating a target gene encoding the endoglycosidase S2 mutant of claim 1 into an expression vector (e.g., pBSYA1S1Z plasmid, pPICZα plasmid, pPIC9K plasmid, pPIC3K plasmid, pPICZA plasmid, pPIC3.5K plasmid, pET22b plasmid, pET28a plasmid, pET30a plasmid, pET32a plasmid, pGEX-6p-1 plasmid, pTrcHis plasmid, pLysS plasmid, pLysE plasmid, pBAD plasmid, pCS plasmid, pGEX-4T-1 plasmid, pET SUMO plasmid, pUC19 plasmid, pBR322 plasmid, pYES2 plasmid, pBV plasmid, pHT01 plasmid, and pHT254 plasmid) to construct a recombinant vector;
2) transforming the recombinant vector into a host cell (e.g., *Pichia pastoris* cell, *Escherichia coli* cell, *Saccharomyces cerevisiae* cell, insect ovary cell, *Bacillus subtilis* cell) to obtain a recombinant cell; and
3) expressing the target gene in the recombinant cell to obtain the endoglycosidase S2 mutant, preferably, the method further comprises step 4): isolating and purifying the endoglycosidase S2 mutant expressed in step 3).

3. A polynucleotide encoding the endoglycosidase S2 mutant of claim 1, preferably, the polynucleotide comprises a sequence as set forth in SEQ ID NO: 7, SEQ ID NO: 8, or SEQ ID NO: 9.

4. A recombinant vector comprising the polynucleotide of claim 3.

5. A host cell transformed with the recombinant vector of claim 4, wherein the host cell is a prokaryotic cell or a eukaryotic cell, preferably selected from *Pichia pastoris* cell, *Escherichia coli* cell, *Saccharomyces cerevisiae* cell, insect ovary cell, and *Bacillus subtilis* cell.

6. Use of the endoglycosidase S2 mutant in antibody glycosylation modification,
wherein, the glycosylation modification includes hydrolysis of a glycosyl group in an antibody, transglycosylation of an antibody, and both,
for example, the glycosylation modification includes preparing an antibody-drug conjugate using an antibody and a disaccharide-small molecule drug conjugate in a one-step method,
wherein, the disaccharide-small molecule drug conjugate is represented by Formula III below: 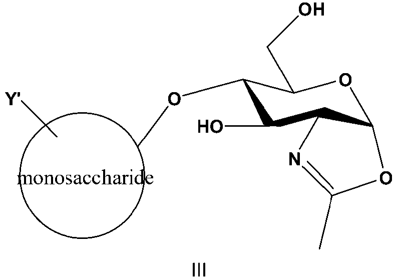 in Formula III, 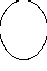 represents a monosaccharide selected from galactose, mannose, and glucose, with its C5 position replaced by Y', and the bond between the two monosaccharides is a 1,4 glycosidic bond,
Y' is further represented by 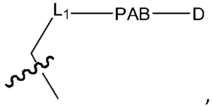
wherein, L₁ represents a dipeptide linker, for example, -Val-Cit-, -Val-Ala-;
PAB represents a p-aminophenylethanol linker;
D represents a small molecule drug moiety, for example, D may be MMAE, MMAF, or maytansine;
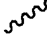 indicates a linking position with the monosaccharide.
alternatively, the glycosylation modification comprises: preparing an antibody-drug conjugate by a two-step method using an antibody and a sugar substrate of Formula II below: 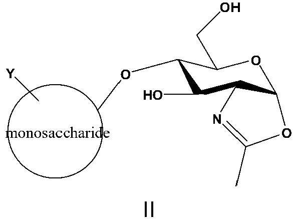 in Formula II, 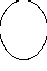 represents a monosaccharide selected from galactose, mannose, and glucose, with its C5 position replaced by Y, and the bond between the two monosaccharides is a 1,4 glycosidic bond,
wherein Y is selected from: 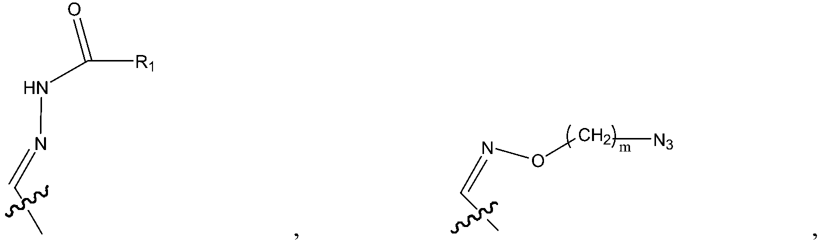 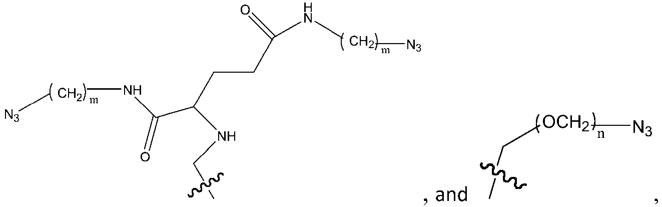
wherein R₁ represents C1-C7 alkyl, preferably C2-C6 alkyl, preferably C3-C5 alkyl, and preferably C3-C4 alkyl, such as methyl, ethyl, propyl, butyl, pentyl, hexyl, and heptyl;
m represents an integer from 1 to 6, for example, m is 1, 2, 3, 4, 5, or 6;
n represents an integer from 1 to 24, for example, n is 1, 2, 3, 4, 5, 6, 7, 8, 9, 10, 11, 12, 13, 14, 15, 16, 17, 18, 19, 20, 21, 22, 23, or 24;
L₁ represents a dipeptide linker, for example, -Val-Cit- or -Val-Ala-;
PAB represents a p-aminophenylethanol linker;
D represents a small molecule drug moiety, for example, D may be MMAE or MMAF;
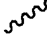 indicates the linking position with the monosaccharide.

7. A method for glycosylation modification of an antibody, the method comprises the step of using the endoglycosidase S2 mutant of claim 1 during a glycosylation modification process.

8. The Use according to claim 6 or the method according to claim 7, wherein the antibody is an antibody having an glycosylation site,
preferably, the antibody is an antibody having an N-glycosylation site,
more preferably, the antibody is a bi-antennary antibody having an N-glycosylation site.
most preferably, the antibody is IgG with a conserved N-glycosylation site at N297 in the Fc region,
specifically, the antibody is a monoclonal antibody, a polyclonal antibody, a bifunctional antibody, a trifunctional antibody, a nanobody fused with an Fc domain, a therapeutic antibody or a functional antibody from a different species,
specifically, the antibody is a human antibody, a murine antibody, or a chimeric antibody; specifically, the antibody is IgG1, IgG2, or IgG4;
specifically, the target of the antibody includes HER2, Claudin 18.2, EGFR, c-Met, NECTIN4, CD276, HER3, CD3, FOLR1, BCMA, CD20, DLL3, MUC1, PD-L1, ROR1, TF, CD19, CD22, CD30, CD70, CD79B, FGFs, MSLN, NT5E, TNFα, CD147, CD24, CD38, CD47, CDH3, CDK4, CDK6, CEACAM5, CLDN6, CTLA4, DDR1, DR5, FAPα, FGFR3, GPRC5D, GR, HLA-DR, ICAM1, IL2R, MELTF, ROR2, TPBG(5T4), VTCN1, ZIP6, CD33, CD25, RSV, VEGF, RANKL, VEGFR2, CTLA-4, CD52, CD319, PD-1, CD274, IgE, IL-6, IL-12, IL-2, C5, IL-17A, CD25, SLAMF7, F10, factor IXa, HAb18G, PCSK9, BlyS, IL23, α4β7, IL-4R-α, HAE, FGF23, and IL6R; preferably, the target of the antibody includes HER2, CD20, EGFR, and PD-1;
specifically the antibody includes trastuzumab, rituximab, pertuzumab, panitumumab, toripalimab, and nivolumab.

9. The method according to claim 7, wherein the method comprises steps of:
1) incubating an antibody with the aforementioned endoglycosidase S2 mutant to hydrolyze the glycosyl group on the antibody ; and/or
2) performing glycosylation modification on the antibody using a sugar substrate under the transglycosylation action of the endoglycosidase S2 mutant,
wherein,
the sugar substrate is a disaccharide represented by Formula I below: 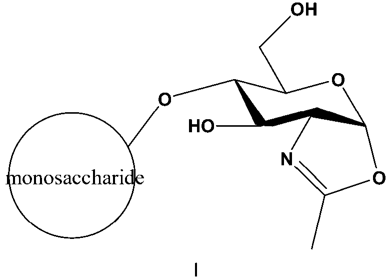 wherein, 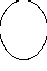 represents a monosaccharide selected from galactose, mannose, and glucose, and the bond between the two monosaccharides is a 1,4 glycosidic bond;
or the sugar substrate is a disaccharide derivative represented by Formula II below: 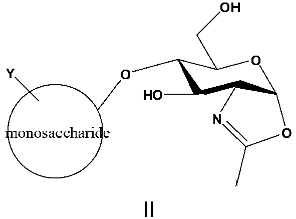 in Formula II, 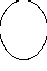 represents a monosaccharide selected from galactose, mannose, and glucose,
with its C5 position replaced by Y,
wherein Y is selected from: 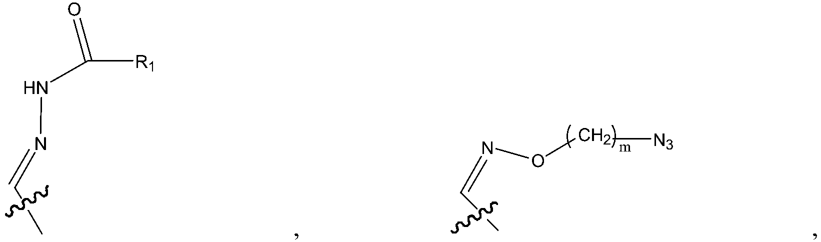 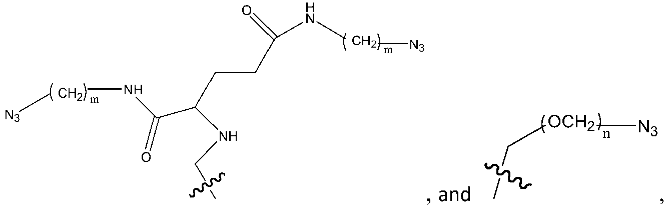
wherein R₁ represents C1-C7 alkyl, preferably C2-C6 alkyl, preferably C3-C5 alkyl, and preferably C3-C4 alkyl, such as methyl, ethyl, propyl, butyl, pentyl, hexyl, and heptyl;
m represents an integer from 1 to 6, for example, m is 1, 2, 3, 4, 5, or 6;
n represents an integer from 1 to 24, for example, n is 1, 2, 3, 4, 5, 6, 7, 8, 9, 10, 11, 12, 13, 14, 15, 16, 17, 18, 19, 20, 21, 22, 23, or 24;
L₁ represents a dipeptide linker, for example, -Val-Cit- or -Val-Ala-;
PAB represents a p-aminophenylethanol linker;
D represents a small molecule drug moiety, for example, D may be MMAE or MMAF;
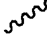 indicates the linking position with the monosaccharide;
or the sugar substrate is a disaccharide-small molecule drug conjugate represented by Formula III below: 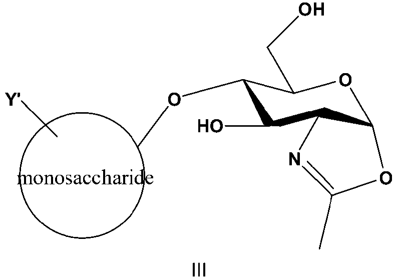 in Formula III, 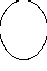 represents a monosaccharide selected from galactose, mannose, and glucose, with its C5 position replaced by Y',
Y' is represented by 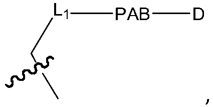
wherein, L₁ represents a dipeptide linker, for example, -Val-Cit-, -Val-Ala-;
PAB represents a p-aminophenylethanol linker;
D represents a small molecule drug moiety, for example, D may be MMAE or MMAF;
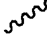 indicates the linking position with the monosaccharide,
specifically, the sugar substrate is selected from: 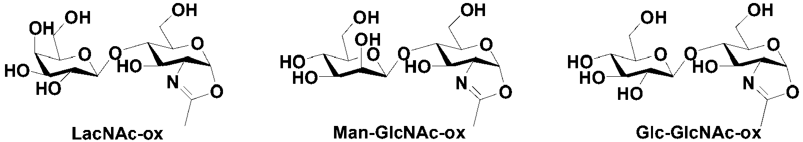 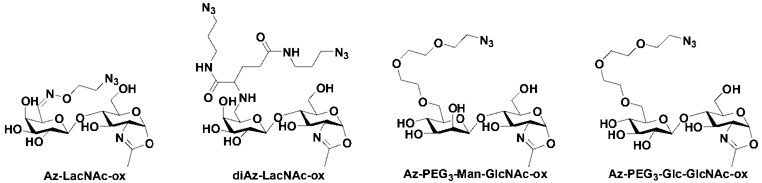 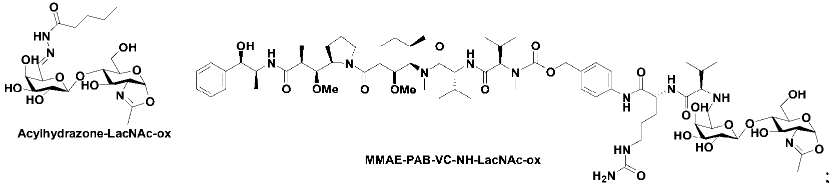
or the sugar substrate is a sugar substrate of the above Formula I, II, or III, of which the oxazoline ring is in an open-ring form;
preferably, the method further comprises the step of reacting the glycosylated antibody modified with the sugar substrate represented by Formula II with a drug-linker to prepare an antibody-drug conjugate, preferably, the drug-linker contains a functional group of hydroxylamine, for example, the drug-linker is NH₂O-VC-PAB-MMAE.
